(19) Europäisches Patentamt
European Patent Office
Office européen des brevets

(11) Veröffentlichungsnummer: **0 034 366**
**B1**

(12) # EUROPÄISCHE PATENTSCHRIFT

(45) Veröffentlichungstag der Patentschrift: **27.06.84**

(51) Int. Cl.³: **C 07 J 41/00**

(21) Anmeldenummer: **81101117.0**

(22) Anmeldetag: **17.02.81**

(54) **Verfahren zur Herstellung von Steroid-20-Carbamatverbindungen.**

(30) Priorität: **19.02.80 US 122398**

(43) Veröffentlichungstag der Anmeldung: **26.08.81 Patentblatt 81/34**

(45) Bekanntmachung des Hinweises auf die Patenterteilung: **27.06.84 Patentblatt 84/26**

(84) Benannte Vertragsstaaten: **AT BE CH DE FR GB IT LI LU NL SE**

(56) Entgegenhaltungen: **ORGANIC REACTIONS, Band III, 1947, Kapitel 7, Seiten 267-286 Ausg. John Wiley and Sons, Inc. New York, U.S.A. E.S. WALLIS et al.: "The Hofmann reaction"**

(73) Patentinhaber: **HENKEL CORPORATION, 7900 West 78th Street, Minneapolis Minnesota 55435 (US)**

(72) Erfinder: **Krbechek, Leroy O., 2041 Orkla Drive, Minneapolis Minnesota 55427 (US)**

(74) Vertreter: **Fues, Johann Friedrich, Dr. et al, Deichmannhaus am Hauptbahnhof, D-5000 Köln 1 (DE)**

**EP 0 034 366 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft die Herstellung bestimmter Steroid-20-Carbamatverbindungen durch chemische Transformation, wobei als Ausgangsmaterialien großtechnisch zugängliche 17-C-Seitenketten-Steroidsubstrate eingesetzt werden können. Die nach dem erfindungsgemäßen Verfahren erhältlichen Carbamate sind wertvolle Zwischenprodukte auf dem Weg der Synthese von Progesteron, Dehydroprogesteron und verwandten Verbindungen, die insbesondere auf pharmazeutischem Gebiet in vielfältiger Weise Verwendung finden können.

Es ist eine Mehrzahl von Vorschlägen gemacht worden, Progesteron und verwandte Verbindungen herzustellen. Erfindungsgemäß werden die dem Progesteron bzw. seinen Homologen entsprechenden 20-Carboxamidoverbindungen als Ausgangsmaterialien eingesetzt. Diese Ausgangsmaterialien sind heute nach wohlfeilen Verfahren großtechnisch zugänglich. So beschreibt die veröffentlichte europäische Patentanmeldung 004 913 ein mikrobiologisches Verfahren, bei dem 3-Oxo-pregna-4-en-20-carbonsäure($\Delta^{4}$-BNC) und/oder 3-Oxo-pregna-1,4-dien-20-carbonsäure($\Delta^{1,4}$-BNC) durch mikrobiellen Seitenkettenabbau an 17-C-Seitenketten-Steroidsubstraten natürlichen Ursprungs gewonnen werden. In der europäischen Patentanmeldung 81 100 146.0 (33 439) wird ein entsprechendes mikrobiologisches Verfahren zur Herstellung der 3-Oxo-pregna-1,4,17(20)-trien-20-carbonsäure($\Delta^{1,4,17}$-BNC) geschildert.

Die US-PS 3 924 933 schildert die mikrobiologische Herstellung der 3-Oxo-pregna-4,17(20)-dien-20-carbonsäure($\Delta^{4,17}$-BNC). Die Strukturformeln der hier genannten BNC-Verbindungen entsprechen den folgenden Formelbildern A bis D mit der Maßgabe, daß der Rest X aus diesen Formeln der Carboxylgruppe-COOH entspricht. Die Struktur der $\Delta^{1,4}$-BNC entspricht A, B stellt $\Delta^{4}$-BNC dar. C beschreibt die $\Delta^{1,4,17}$-BNC, während D schließlich die $\Delta^{4,17}$-BNC wiedergibt.

A

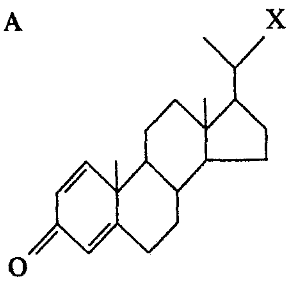

B

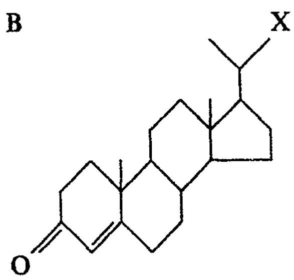

C

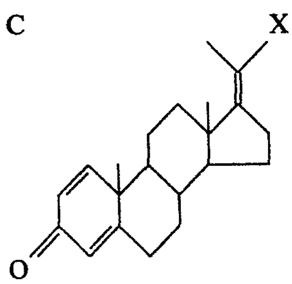

D

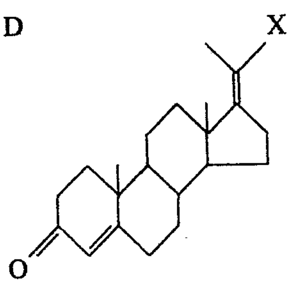

Zur Umwandlung dieser Ausgangsmaterialien zum Progesteron bzw. Verbindungen vom Progesterontyp muß der Substituent X in 20-Stellung zur 20-Oxogruppe umgewandelt werden. Die Erfindung schildert einen wichtigen Teilschritt dieser chemischen Transformation.

BNC-Verbindungen gemäß A bis D können durch Halogenierung — vorzugsweise mit Thionylchlorid — in die entsprechenden Säurechloride umgewandelt werden, d. h. in Verbindungen, in denen X dem Rest —COCl entspricht. Diese Säurechloride werden durch ammoniakalische Behandlung in die entsprechenden Säureamide umgewandelt, d. h. in die Verbindungen A bis D, in denen X dem Rest $-CONH_2$ entspricht. Einzelheiten zu diesen Verfahrensschritten finden sich in der älteren europäischen Patentanmeldung 81 100 145.2 (34 248) sowie in der parallelen europäischen Patentanmeldung 81 101 114.7 (34 364) (»Neue 20-substituierte 3-Oxo-pregna-4-en-steroidverbindungen und Verfahren zu ihrer Herstellung«, U.S.-Priorität vom 19. Februar 1980) der gleichen Anmelderin.

Erfindungsgemäß werden die Säureamide zunächst einer N-Bromierung unterworfen (X = —CONHBr), dann wird unter Abspaltung von Bromwasserstoff und intermediärer Isocyanatbildung (X = —NCO) das Carbamat gebildet (X = —NHCOOR).

Gegenstand der Erfindung ist dementsprechend ein Verfahren zur Herstellung von Steroid-20-Carbamaten aus der Gruppe 3-Oxo-pregn-4-en-20-carbamat, 3-Oxo-pregna-1,4-dien-20-carbamat, 3-Oxo-pregna-4,17(20)-dien-20-carbamat und/oder 3-Oxo-pregna-1,4,17(20)-trien-20-carbamat, wobei dieses Verfahren dadurch gekennzeichnet ist, daß man die entsprechenden Steroid-20-Carboxamide einer Transhalogenierung mit N-Bromsuccinimid oder N-Bromphthalsäureimid unterwirft, das Reaktionsprodukt mit einer starken Base behandelt und mit einem einwertigen Alkohol umsetzt. Vorzugsweise wird dieses Mehrstufenverfahren in einem Reaktionsschritt ohne Isolierung der Reaktionszwischenprodukte durchgeführt.

Die N-Halogenierung der erfindungsgemäß als Ausgangsmaterial eingesetzten Carboxamide erfolgt durch Transhalogenierung mittels ausgewählter N-Haloimidverbindungen. Die erfindungsgemäß bevorzugten Transhalogenierungsmittel sind N-Bromsuccinimid und N-Bromphthalimid. Prinzipiell — wenn auch weniger bevorzugt — läßt sich die Reaktion auch mit den entsprechenden Chloriden und gewünschtenfalls auch den entsprechenden Fluoriden und Iodiden durchführen.

Das als Reaktant eingesetzte N-Haloimid wird wenigstens in einer der eingesetzten Steroidverbindung äquivalenten Menge verwendet. Vorzugsweise wird jedoch ein mäßiger Überschuß der N-Haloimidverbindung eingesetzt. Üblicherweise werden diese Halogenierungsmittel in Mengen von 1,1 bis 5,0 Aquivalenten, vorzugsweise von etwa 1,2 bis 3,0 Äquivalenten je Äquivalent Steroidverbindung verwendet.

Zweckmäßigerweise wird weiterhin diese Umsetzung in Gegenwart von Lösungsmitteln durchgeführt. Hierbei können aprotische Lösungsmittel Verwendung finden, die es erlauben, die intermediär entstehenden Steroid-N-Haloimidverbindungen als solche zu isolieren. Geeignete aprotische Lösungsmittel sind beispielsweise Halogenkohlenwasserstoffe, wie Methylenchlorid oder Äthylendichlorid. Hinreichende Mengen des Lösungsmittels sollten Verwendung finden, um die Umsetzung der sonst zähflüssigen Reaktionsmasse zu erleichtern. In der bevorzugten, im folgenden geschilderten Ausführungsform werden jedoch als Lösungsmittel oder wenigstens als Teil eines Lösungsmittelgemisches Alkohole eingesetzt, die dann die unmittelbare Bildung der Carbamate im Reaktionsansatz ermöglichen.

Die Verfahrenstemperatur liegt zweckmäßigerweise im Bereich von 0° C bis 95° C, insbesondere im Bereich von etwa 10° C bis 60° C.

Zur weiteren Umwandlung der intermediär gebildeten N-Halogen-substituierten Steroidamide werden diese mit starken Basen behandelt. Als starke Basen werden vorzugsweise Natriumhydroxid, Kaliumhydroxid bzw. die entsprechenden Alkoxide, insbesondere Natriummethoxid eingesetzt. Die basische Verbindung wird wenigstens in äquivalenten Mengen, bezogen auf vorliegende Steroidverbindung, verwendet, wobei zweckmäßigerweise mit einem gewissen Überschuß der stark-basischen Komponente gearbeitet wird. Nach Verfahrensabschluß kann dieser Basenüberschuß durch Neutralisieren mit Säuren, beispielsweise Essigsäure, beseitigt werden.

Die erfindungsgemäß angestrebte Carbamatverbindung entsteht durch Umsetzung des jetzt vorliegenden Reaktionszwischenproduktes mit einem einwertigen Alkohol, der vorzugsweise nicht mehr als 10 C-Atome enthält. Bevorzugt wird mit niederen Alkanolen als Alkoholkomponente gearbeitet und insbesondere mit solchen bis zu 5 C-Atomen. Der Alkohol kann, wie eingangs dargestellt, bereits von Anfang an als reaktives Lösungsmittel vorgelegt werden, so daß erfindungsgemäß auf diesem Wege in einem Zuge das Steroidcarbonsäureamid in das Carbamat umgewandelt wird. Es kann zweckmäßig sein, mit einem Lösungsmittelgemisch zu arbeiten, das die reaktive Alkoholkomponente in Abmischung mit einer nichtreaktiven Lösungsmittelkomponente — beispielsweise den bereits genannten halogenierten Kohlenwasserstoffen — enthält. Geeignet sind beispielsweise Alkohol-/Halogenkohlenwasserstoff-Gemische in einem Gewichtsverhältnis von etwa 15 : 1 bis etwa 1 : 10. Die Alkoholkomponente ist auf jeden Fall in stöchiometrisch hinreichenden Mengen einzusetzen, um die Carbamatbildung zu ermöglichen. Geeignete Alkohole sind beispielsweise Methanol, Ethanol, n-Propanol, 2-Propanol, n-Butanol, 2-Butanol, tert.-Butanol. Aber auch Alkohole mit einer größeren Kohlenstoffzahl und/oder einer anderen Struktur können im erfindungsgemäßen Verfahren eingesetzt werden. Als Beispiele seien benannt: Benzylalkohol, 2-Alkoxyalkanole wie 2-Methoxyäthanol, sowie Mischungen der hier genannten Komponenten.

Erfindungsgemäß wird es bevorzugt, die Reaktion in Abwesenheit von Wasser oder wenigstens unter Einschränkung eines eventuell vorliegenden Wassergehalts durchzuführen. Der praktisch vollständige Ausschluß von Wasser ist erforderlich, wenn Steroidverbindungen mit einer Doppelbindung in 17(20)-Stellung als Ausgangsmaterial eingesetzt werden. Bei den anderen Komponenten wurde überraschenderweise festgestellt, daß Wasser bis zu etwa 5 Gew.-%, vorzugsweise in Mengen von nicht mehr als etwa 2,5 Gew.-%, bezogen auf das Gewicht der Reaktanten, toleriert werden kann.

Es ist weiterhin bevorzugt, die Umsetzungen in inerter Atmosphäre, insbesondere unter Stickstoff, durchzuführen und das Reaktionsgefäß vor Durchführung der Reaktion in entsprechender Weise hinreichend auszuspülen. Zur Beseitigung von Feuchtigkeitsspuren, beispielsweise im Lösungsmittel bzw. Lösungsmittelgemisch, kann Calciumhydrid besonders geeignet sein.

Es kann weiterhin zweckmäßig sein, dem Reaktionsgemisch einen Urethanbildungskatalysator, beispielsweise Dibutylzinndilaurat, zuzusetzen.

Im erfindungsgemäßen Verfahren kann es zweckmäßig sein, dem Reaktionsgemisch nach der Bildung des Steroid-N-Haloamids ein Reduktionsmittel zuzusetzen. Ein geeignetes Reduktionsmittel in diesem Sinne ist Natriumthiosulfat.

Verschiedenartigste Transformationsreaktionen an Steroidverbindungen sind im Stand der Technik beschrieben worden. Keiner dieser Vorschläge schildert jedoch das erfindungsgemäße Verfahren zur Herstellung der Steroid-20-Carbamatverbindungen. Diese Carbamatverbindungen können ihrerseits wichtige Ausgangsmaterialien für die letztendlich angestrebte Ausbildung der 20-Oxogruppe sein. So kann die Carbamatfunktion in die 20-Aminofunktion umgewandelt werden, die ihrerseits wieder zur 20-Oxogruppe umgewandelt werden kann. Näheres zu diesen möglichen nachfolgenden Verfahrensschritten findet sich beispielsweise in den parallelen europäischen Patentanmeldungen 81 101 118.8 (34 367) (»Verfahren zur hydrolytischen Spaltung von Steroid-20-Carbamaten«, U.S.-Priorität vom 19. Februar 1980), 81 101 113.9 (34 363) (»Neue Steroidverbindung mit einer 20-Amino-Funktion und Verfahren zu ihrer Herstellung«, U.S.-Prioritäten vom 19. Februar 1980), 81 101 115.4 (34 365) (»Verfahren zur Herstellung von 3,20-Dioxo-pregn-4-en und/oder 3,20-Dioxo-pregna-1,4-dien«, U.S.-Priorität vom 19. Februar 1980) der gleichen Anmelderin.

Aus dem Stand der Technik zur chemischen Transformation von Steroidverbindungen wird verwiesen auf Canadian Journal of Chemistry, Band 34 (1956), S. 982—990, sowie Helvetica Chimica Acta, Band XXXII, Teil VI (1949), Nr. 255, S. 1922—1933, sowie a.a.O. Band XXXII, Teil V (1949), Nr. 233, S. 1764—1769. Verwiesen wird weiterhin auf JACS, Band LXX (1948), Nr. 3, S. 887—892, sowie Helvetica Chimica Acta, Band XXXII, Teil V (1949), Nr. 232, S. 1758—1763. Verwiesen sei schließlich auch noch auf die US-PS 3 519 658.

## Beispiel

1 kg 3-Oxo-pregn-4-en-20-carbonsäureamid wird zu 4,5 l Methanol gegeben. Das Methanol ist zuvor mit Calciumhydrid getrocknet worden. In das Reaktionsgemisch werden weiterhin 375 g Natriummethoxid eingegeben. Die Reaktionsmischung wird dann unter Stickstoff auf 45°C erwärmt. 600 g handelsübliches N-Bromsuccinimid werden dem Reaktionsgemisch bei 45°C unter Stickstoff zugesetzt. Die Mischung wird für einen Zeitraum von etwa 45 Minuten gerührt, die Stickstoffatmosphäre bleibt dabei aufrechterhalten.

Die Reaktionslösung wird anschließend mit 2 l Eisessig angesäuert. Der nach Entfernung des Lösungsmittels unter verringertem Druck verbleibende Rückstand wird in Methylenchlorid gelöst und intensiv mit Wasser, Natriumthiosulfat und erneut Wasser gewaschen.

Anschließend wird das Methylenchlorid unter verringertem Druck entfernt. Es werden 1200 g Rohprodukt erhalten. Die Analyse des Rohprodukts zeigt, daß sich das 3-Oxo-pregn-4-en-20-methylcarbamat in einer Ausbeute von mehr als 80% der Theorie gebildet hat. Im Reaktionsrohprodukt wird nur noch ein geringer Rest des eingesetzten Säureamids gefunden.

Die Ausbeute an gewünschtem Reaktionsprodukt wird durch den Zusatz von Dibutylzinndilaurat leicht verbessert.

Praktisch gleiche Verfahrensergebnisse werden erhalten, wenn anstelle des N-Bromsuccinimids N-Bromphthalimid eingesetzt wird.

Wird 3-Oxo-pregna-1,4-dien-20-carbonsäureamid als Ausgangsmaterial in gleicher Menge und unter gleichen Verfahrensbedingungen eingesetzt, so fällt auch hier das entsprechende 3-Oxo-pregna1,4-dien-20-methylcarbamat in einer Ausbeute über 80% der Theorie an.

## Patentansprüche

1. Verfahren zur Herstellung von Steroid-20-Carbamaten aus der Gruppe 3-Oxo-pregn-4-en-20-carbamat, 3-Oxo-pregna-1,4-dien-20-carbamat, 3-Oxo-pregna-4,17(20)-dien-20-carbamat und/oder

3-Oxo-pregna-1,4,17(20)-trien-20-carbamat, dadurch gekennzeichnet, daß man die entsprechenden Steroid-20-Carboxamide einer Transhalogenierung mit N-Bromsuccinimid oder N-Bromphthalimid unterwirft, das Reaktionsprodukt mit einer starken Base behandelt und mit einem einwertigen Alkohol umsetzt.

2. Verfahren nach Anspruch 2, dadurch gekennzeichnet, daß man das Mehrstufen-Verfahren in einem Zuge ohne Isolierung der Reaktionszwischenprodukte durchführt.

3. Verfahren nach Ansprüchen 1 und 2, dadurch gekennzeichnet, daß man mit Alkoholen mit bis zu 10 C-Atomen, insbesondere mit niederen Alkanolen mit vorzugsweise bis zu 5 C-Atomen als Alkoholkomponente arbeitet.

4. Verfahren nach Ansprüchen 1 bis 3, dadurch gekennzeichnet, daß man in Gegenwart von Lösungsmitteln arbeitet, wobei bevorzugt der zur Carbamatbildung benötigte Alkohol — ggf. in Abmischung mit inerten Lösungsmitteln, wie Halogenkohlenwasserstoffen — als reaktives Lösungsmittel eingesetzt werden kann.

5. Verfahren nach Ansprüchen 1 bis 4, dadurch gekennzeichnet, daß man als starke Base Natriumhydroxid, Kaliumhydroxid und/oder die entsprechenden Alkoxide einsetzt.

6. Verfahren nach Ansprüchen 1 bis 5, dadurch gekennzeichnet, daß man bei Temperaturen von 0° C bis 95° C, insbesondere im Bereich von etwa 10° C bis 60° C und vorzugsweise in inerter Atmosphäre arbeitet.

7. Verfahren nach Ansprüchen 1 bis 6, dadurch gekennzeichnet, daß man dem Reaktionsgemisch einen Urethanbildungskatalysator zusetzt.

**Claims**

1. A process for preparing a steroid-20-carbamate compound selected from the group consisting of 3-oxo-pregn-4-ene-20-carbamate, 3-oxo-pregna-1,4-diene-20-carbamate, 3-oxo-pregna-4,17(20)-diene-20-carbamate, and/or 3-oxo-pregna-1,4,17(20)-triene-20-carbamate, characterized in that the respective steroid-20-carboxamides are subjected to a transhalogenation with N-bromosuccinimide or N-bromophthalimide, the reaction product is treated with a strong base and is reacted with a monohydric alcohol.

2. The process according to claim 1, characterized in that the multi-stage process is carried out nonstop without isolating the intermediate reaction products.

3. The process according to claims 1 and 2, characterized in that alcohols having up to 10 carbon atoms, more particularly lower alkanols having preferably up to 5 carbon atoms, are used as the alcohol component.

4. The process according to claims 1 to 3, characterized in that it is carried out in the presence of solvents, in which process preferably the alcohol required for the carbamate formation — optionally in admixture with inert solvents, such as halocarbons — can be used as a reactive solvent.

5. The process according to claims 1 to 4, characterized in that sodium hydroxide, potassium hydroxide, and/or the corresponding alkoxides, are used as the strong base.

6. The process according to claims 1 to 5, characterized in that there is operated at a temperature of from 0° C to 95° C, and more specifically in the range from 10° C to 60° C, and preferebly in an inert atmosphere.

7. The process according to claims 1 to 6, characterized in that an urethane-formation catalyst is added to the rection mixture.

**Revendications**

1. Procédé de préparation de 20-carbamate-stéroïdes du groupe 3-oxo-pregn-4-ène-20-carbamate, 3-oxo-pregna-1,4-diène-20-carbamate, 3-oxo-pregna-4,17(20)-diène-20-carbamate et/ou 3-oxo-pregna-1,4,17(20)-triène-20-carbamate, caractérisé en ce qu'on soumet les 20-carboxamides stéroïdes correspondants à une transhalogénation par du N-bromosuccinimide ou du N-bromophthalimide, qu'on traite le produit de réaction par une base forte et qu'on le fait réagir sur un monoalcool.

2. Procédé selon la revendication 1, caractérisé en ce qu'on réalise le procédé en plusieurs stades en une fois, sans isolation des produits intermédiaires de la réaction.

3. Procédé selon les revendications 1 et 2, caractérisé en ce qu'on travaille avec, en tant que constituant alcool, des alcools ayant jusqu'à 10 atomes de carbone, en particulier des alcanols inférieurs ayant de préférence jusqu'à 5 atomes de carbone.

4. Procédé selon les revendications 1 à 3, caractérisé en ce qu'on travaille en présence de solvants, ce à l'occasion de quoi on peut utiliser de préférence, en tant que solvant réactif, l'alcool nécessaire à la formation du carbamate — éventuellement en mélange avec des solvants inertes comme des hydrocarbures halogénés —.

5. Procédé selon les revendications 1 à 4, caractérisé en ce qu'on utilise en tant que base forte de l'hydroxyde de sodium, de l'hydroxyde de potassium et/ou les alkylates correspondants.

6. Procédé selon les revendications 1 à 5, caractérisé en ce qu'on travaille à des températures de 0 à 95° C, en particulier dans l'intervalle d'environ 10 à 60° C et de préférence en atmosphère inerte.

7. Procédé selon les revendications 1 à 6, caractérisé en ce qu'on ajoute au mélange réactionnel un catalyseur de formation d'uréthane.